# EUROPEAN PATENT APPLICATION

(11) **EP 4 324 370 A1**
(43) Date of publication of application: **21.02.2024**
(21) Application number: 22787674.5
(22) Date of filing: 13.04.2022
(51) Int. Cl.: A47K 10/48

(54) **DEVICE FOR DRYING FEET AND/OR FOOTWEAR**

(30) Priority: 16.04.2021 ES 202130785 U
(71) Applicant: Valiryo Technologies S.L., 31191 Barbatain (Navarra) (ES)
(72) Inventor: ESANDI MATEO, Ion, 31191 Barbatain ( Navarra) (ES); JIMENEZ GARCIA, Andoni, 31191 Barbatain ( Navarra) (ES); RICO JIMENEZ, Carlos, 31191 Barbatain ( Navarra) (ES)
(74) Representative: Cabinet Beau de Loménie
(86) International application number: PCT/ES2022/000018
(87) International publication number: WO 2022/219210

(57) **Abstract**

The present invention relates to a device for drying feet and/or footwear, comprising: a casing (3) with at least one opening for the inflow of air into the casing; an upper surface of the casing for receiving the sole of the foot and/or footwear, comprising at least one lower hole for the outflow of air from inside the casing to the exterior so as to come into contact with the sole of the foot and/or footwear; lateral walls projecting from the upper surface, each lateral wall comprising at least two lateral holes for the outflow of air from inside the casing; and a motor that absorbs air through the air inflow opening in order to expel it through the air outflow holes, wherein the lateral holes are located at least at two different heights with respect to the upper surface of the casing in order to expel the air over the sides and the instep of the foot and/or footwear.

## Description

### Technical field

The present invention is related to devices for drying the human body, more specifically to devices for drying feet and footwear.

### State of the art

Devices for drying feet based on a perforated casing which houses a motor projecting air outward through said holes are currently widely known.

These devices are of particular interest to elderly people or people with reduced mobility who cannot comfortably or safely dry their feet with a towel.

They are also necessary to properly dry footwear in order to avoid the introduction of water into covered spaces and protect the footwear.

In this sense, the Chinese utility model CN201519099U proposes a foot dryer with air outlets both on the upper surface of the casing to dry the lower surface of the foot and on vertical projections that transversely project the air to dry the lateral surfaces of the foot, all of them at the same height with respect to the upper surface of the casing, which does not allow for the proper drying of the entire foot.

This solution optionally incorporates a bridge, the bottom surface of which has air outlets that can dry the top and front of the foot.

The problem with this solution for users with reduced mobility is that this bridge causes difficulties in using the device and poses a high risk of tripping.

Furthermore, the coverage thereof is limited and it cannot adequately dry the top of the foot closest to the ankle.

Therefore, the user must choose between an option with a bridge, which implies a risk of accident, and another option without a bridge, which carries out an incomplete drying.

Another proposal for a device for drying feet can be found in the Japanese patent JPH0813296, which discloses a device for washing and drying feet in which the drying is carried out by projecting air from an elevated position with respect to the foot.

Once again, this solution is incomplete because it lacks means allowing for a comprehensive drying of the foot and furthermore it is difficult to use for users with limited mobility.

In light of the described disadvantages of current solutions, it is clear that it is necessary to find a solution that enables the effective and complete drying of the foot and which, at the same time, is easy to use, posing no risk for users with reduced mobility and lacking elements such as bridges or elevated structures that could cause users to trip when placed in front of their foot.

### Object of the invention

In order to fulfil this aim and solve the aforementioned technical problems, in addition to providing additional advantages that can be subsequently derived, the present invention relates to a foot dryer consisting of a casing inside which a motor is housed, the casing having an opening, which may be a perforated area, through which the motor absorbs air into the casing and subsequently expels it out of the casing through different lower holes located on the upper surface of the casing towards an area on which the user will place their foot. These lower holes direct the air from the casing to the sole of the foot to dry it.

In order for the projection of air to completely dry the foot, the device has a series of lateral holes at different points on the surface thereof, through which air is expelled onto the sides and instep of the foot.

Holes are found on the upper surface of the casing and project air onto the sole of the foot.

Another series of at least two holes are arranged in two lateral walls, raised with respect to the upper surface of the casing, the lateral holes being located at different heights with respect to the upper surface of the casing. In this way, the outflow of air from the lateral holes goes over the corresponding lateral area of the foot and the upper part of the foot, surrounding the foot with the air that comes out of the inside of the casing, thereby completely drying the foot.

These lateral walls have a great advantage in that although they allow the foot to dry completely, even the upper part of the same, they do not go into the space above the foot so they are not an obstacle in the path of the foot, which can move vertically and forward without impacting any protruding element of the device that could cause the user to trip.

The arrangement of the outflow holes at different heights allows for a comprehensive drying of the foot, covering all exposed skin.

The device has presence sensors which, when detecting that an object placed in the proximity of the drying area, activate the motor to start the drying.

In this way, the user only has to place their foot in the drying area, the foot then being detected by the sensors activating the motor and starting the projection of air both towards the bottom of the foot and towards the sides and upper part of the same through the different holes arranged on both the upper surface of the casing and on the aforementioned lateral walls.

The device can incorporate different inflow air cleaning systems, such as a HEPA particle filter, an ozone converter or/and an ultraviolet light emitter, with the function of sanitising and disinfecting the air introduced into the motor, as well as extending the useful life thereof by limiting the inflow of impurities.

These sanitisation systems can also be activated by the sensors detecting the presence of an element in the drying area.

The interior of the casing narrows from the motor area to the air outflow area such that the air increases in speed as the size of the section through which it circulates decreases.

According to an alternative embodiment, the air outflow holes can have the shape of slots and be positioned perpendicular to the movement of the foot along the channel created by the lateral walls, which, together with the high pressure of the motor, allows for drying by dragging the water. Furthermore, the slot-shaped configuration can achieve the same effect as the arrangement of the lateral holes at different heights, since by having the slot span the height from the sole to the top of the foot, the air outflow covers the entire foot to completely dry the same.

To make the device more comfortable for the user to use, the drying platform can optionally have an inclination with respect to the horizontal plane, which facilitates drying the foot both while sitting and standing.

This inclined surface can optionally have a curved configuration that better suits the mobility of the user.

It is also anticipated that the device may incorporate means for emitting infrared light in order to provide heat to the surface to be dried and thus achieve an even better result.

This option is especially useful for users with athlete's foot who require a very meticulous drying.

To further facilitate the use thereof use by users, an embodiment of special interest is proposed, in which at least one lateral air outflow hole in the lateral walls is aligned with at least one lower air outflow hole in the upper surface of the casing, ensuring that a user who cannot see the placement of the lateral holes in the lateral walls, due to the arrangement thereof, is guided by the lower holes the user can see on the upper surface of the casing and is therefore able to carry out an effective and complete drying.

Optionally, the inclusion of ultraviolet light emitters or suitable frequencies to achieve sanitising effects on the surface of the device is envisioned so that, in addition to the drying function, microorganisms can also be eliminated and, therefore, common foot diseases, such as those related to athlete's foot, can be prevented.

Although the description is related to drying feet, as stated in the state of the art, it must be understood that the invention can also be used for drying and disinfecting footwear.

### Description of the figures

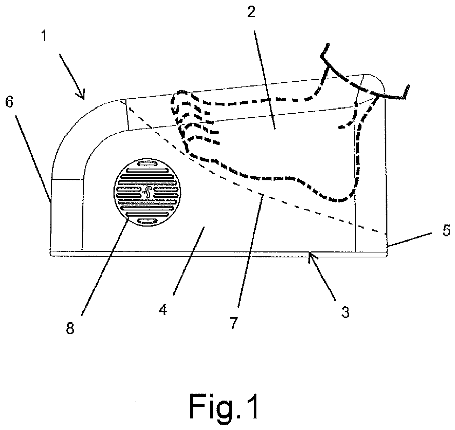

Figure 1 shows a side view of the device as it would be used.
Figure 2 shows a perspective of the object of the invention.
Figure 3 shows a perspective view of the device in which the inside of the casing can be seen by means of dashed lines.
Figure 4 shows a front view of the device of the invention.
Figure 5 shows a cross section of the device showing the narrowing of the casing which favours the speed of the drying air.
Figure 6 shows a variant of the invention for use with the tip of the foot facing towards the front.

### Detailed description of the invention

The present invention relates to a device (1) for drying feet (2) and/or footwear which comprise a casing (3) comprising two lateral surfaces (4), a front (5) and a rear part (6), a drying volume being located on the upper surface (7) of the casing (3) and between two lateral walls (12) arranged projecting from the upper surface (7), such that the tip of the foot (2) is oriented towards the back (6) of the casing (3) in the drying process.

The casing (3) has an opening for the inflow of air (8) into the same and an air outflow through lower holes (9) of the upper surface (7) and lateral holes (9') arranged in the lateral walls (12) that communicate the inside with the exterior of the casing (3) and through which air comes out to dry the foot (2).

A motor (10) located inside the casing (3) in correspondence with the air inflow opening (8) absorbs air from the exterior through this air inflow opening (8) and projects it to the outside through the holes (9, 9').

The motor (10) is preferably arranged in the area closest to the rear part (6) of the casing (3).

In a preferred configuration, the interior of the casing (3) undergoes a progressive narrowing from the area of the motor (10) to the area where the holes (9) are located, allowing air to be driven through the interior thereof such that air expelled from the motor (10) maintains a high speed, given the narrowing of the section. The device (1) has sensors (11) that detect the presence of an object in the drying volume. When an object is detected in this drying volume, the sensors (11) cause the motor (10) to start and the air to flow through the holes (9), and when the detection of the object is interrupted, the motor (10) stops.

To detect the presence of objects with a larger volume, in a preferred embodiment, the device (1) has more than one sensor (11), which are at different heights from each other.

The device (1) has two lateral walls (12) arranged in an eminently vertical projection on the upper surface (7). In said lateral walls (12) there are at least two lateral holes (9'), in communication with the inside of the casing (3), through which the air projected by the motor (10) comes out, said lateral holes (9') being oriented towards the drying volume above the upper surface (7) of the casing, with at least two holes located at different heights with respect to said upper surface (7).

This allows air to be projected from the lateral walls (12) through the lateral holes (9') at different heights, drying both the upper part of the foot (2) and the sides thereof, even drying the part corresponding to the ankles. According to one design option, the lateral walls (12) are not arranged in a completely vertical projection with respect to the upper surface (7), but rather have an inclination, the lateral walls (12) converging towards the drying volume. With this configuration, the holes are oriented towards the top surface of the foot (2), achieving a faster drying.

The lateral walls (12) have a distance between them that allows for a foot (2) to easily pass between the same, with the minimum distance between the columns (12) being at least 10 cm.

Since the complete drying of the foot (2) occurs by the outflow of air at different heights, there is no need for a structure above the foot (2) to dry the upper part thereof, and thus the complete drying of the foot (2) can be carried out with a device (1) that lacks raised elements that could trip the user.

The user simply has to move their foot back and forth to achieve complete drying. The user can insert their foot (2) through the front (5), through the back (6) or even vertically.

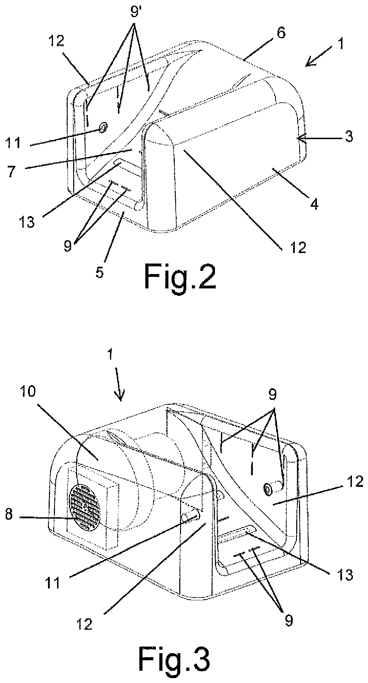

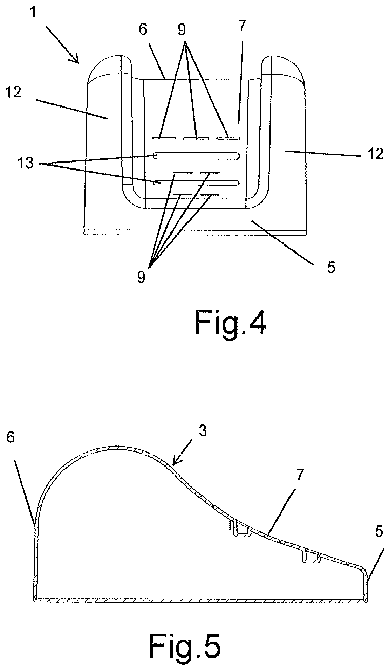

Both the lateral holes (9') and the lower holes (9) can have a particular slot-shaped configuration, as can be seen in Figures 2 to 4, which allows the air outflow to have the form of a curtain, thereby dragging of the water on the foot (2) with the forward and backward movement thereof, thus optimising the drying thereof.

The lateral walls (12) can have a constant height with respect to the upper surface (7) or have a different configuration, in which one part is higher, and therefore they may have greater number of holes (11), or the same with a greater size, such as in the area in which the heel is envisaged and fewer where the toe (2) is envisaged.

In this way, energy use is optimised by applying the necessary amount of air to completely dry to each part.

Ideally, the user will insert their foot (2) so that the toe is towards the back part (6) and their heel is towards the front (5). In this case the columns (11) will have a greater height with respect to the upper surface (7) in the area closest to the front (5). This is an optimal configuration for use while standing.

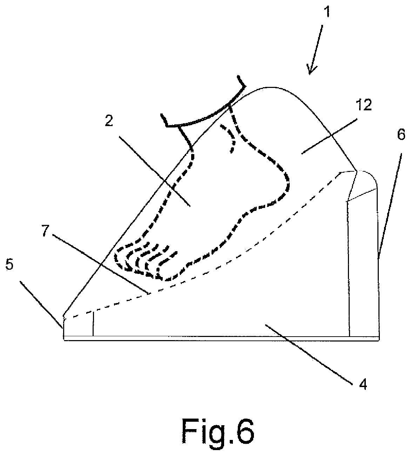

In another configuration, such as the one represented in Figure 6, it is envisaged that the lateral walls (12) have a greater height with respect to the upper surface (7) in the area closest to the rear part (6), and a lesser height in the front (5). This configuration is optimal for the user to place their heel closest to the rear (6) and the tip of their foot (2) closest to the front (5) and is especially ergonomically suitable for use while seated, as it is only necessary to bend one's knee. In this case the rear part (6) would be the access area for the foot (2).

On the air inlet of the motor (10), elements can optionally be placed to clean the air inflow, such as a HEPA filter, an ozone converter or/and an ultraviolet light emitter.

For better ergonomic features of the device (1) to make the use thereof more comfortable for users, as well as to favour water evacuation, the upper surface (7) may optionally be inclined less than 60° with respect to the horizontal plane, the optimum inclination being between 25° and 50° with the rear part (6) being elevated with respect to the front (5).

Likewise, the upper surface (7) can have a concave curved shape that corresponds to the natural movement of the foot (2) with respect to the knee or hip, maintaining the same distance with respect to the upper surface (7) of the casing (3) to the foot (2) throughout the entire length thereof.

The device (1) can incorporate an infrared light source with the objective of heating the foot (2), which is oriented towards the drying volume.

According to one embodiment of the invention, at least one lateral hole (9') of the columns (12) is aligned with at least one lower hole (9) of the upper surface (7). This allows the user to know where the lower holes (9) of the upper surface (7) are located by observing where the lateral holes (9') of the lateral walls (12) are located even though the foot (2) covers the upper surface (7).

According to another embodiment of the invention, the shortest distance between the lateral walls (12) is greater than or equal to 10 cm, which allows for comfortable use by the interested party and reduces the chances of tripping.

Optionally, the device (1) can incorporate ultraviolet radiation lamps (13) or radiation lamps with other sanitising frequencies, with the aim of disinfecting the foot (2) in an action that may or may not be simultaneous with the intended drying, the ultraviolet radiation lamps (13) preferably able to be arranged in relation to the upper surface (7) of the device (1) and activated by the sensors (11) when they detect the proximity of an element.

## Claims

1. A device (1) for drying feet (2) and/or footwear, which comprises:
- a casing (3) with at least one opening (8) for the inflow of air into the casing (3);
- an upper surface (7) of the casing (3) for receiving the sole of the foot (2) and/or footwear comprising at least one lower hole (9) for the outflow of air from inside the casing (3) so as to come into contact with the sole of the foot (2) and/or footwear;
- lateral walls (12) projecting from the upper surface (7), each lateral wall (12) comprising at least two lateral holes (9') for the outflow of air from inside the casing (3)
- and a motor (10) that absorbs air through the air inflow opening (8) in order to expel it through the air outflow holes (9, 9'),
**characterised in that** the lateral holes (9') are located at least at two different heights with respect to the upper surface (7) of the casing (3) to expel the air over the sides and the instep of the foot (2) and/or footwear.

2. The device (1) for drying feet (2) and/or footwear according to the previous claim, **characterised in that** it comprises at least one sensor (11) to detect the presence of the foot (2) and/or footwear.

3. The device (1) for drying feet (2) and/or footwear according to the previous claim, **characterised in that** the shortest distance between the lateral walls (12) is greater than or equal to 10 cm.

4. The device (1) for drying feet (2) and/or footwear according to any one of the previous claims, **characterised in that** the upper surface (7) of the casing (3) has a concave curved shape.

5. The device (1) for drying feet (2) and/or footwear according to any one of the previous claims, **characterised in that** the interior of the casing (3) undergoes a progressive narrowing from the area of the motor (10) to the air outflow holes (9, 9').

6. The device (1) for drying feet (2) and/or footwear according to any one of the preceding claims, **characterised in that** it comprises elements for cleaning the inflowing air between the air inflow opening (8) and the motor (10).

7. The device (1) for drying feet (2) and/or footwear according to any one of the previous claims, **characterised in that** at least one of the air outflow holes (9, 9) is slot-shaped.

8. The device (1) for drying feet (2) and/or footwear according to any one of the previous claims, **characterised in that** the upper surface (7) has an inclination between 1° and 60° with respect to the horizontal plane.

9. The device for drying feet (2) and/or footwear according to the previous claim, **characterised in that** the inclination of the upper surface (7) is between 25° and 50° with respect to the horizontal plane.

10. The device (1) for drying feet (2) and/or footwear according to any one of the previous claims, **characterised in that** it comprises at least one infrared light source aimed at the foot (2) and/or footwear.

11. The device (1) for drying feet (2) and/or footwear according to any one of the previous claims, **characterised in that** it comprises at least one source of ultraviolet radiation (15) or other radiation with another frequency that also has a sanitising effect aimed at the foot (2) and/or footwear.
